# EUROPÄISCHE PATENTANMELDUNG

(11) **EP 2 548 882 A1**
(43) Veröffentlichungstag der Anmeldung: **23.01.2013**
(21) Anmeldenummer: 11194048.2
(22) Anmeldetag: 16.12.2011
(51) Int. Cl.: C07K 7/08, C07K 14/245

(54) **Verfahren zur rekombinanten Herstellung von mehrfach Disulfid-verbrückten Peptiden**

(30) Priorität: 07.01.2011 DE 102011008075
(71) Anmelder: ALNuMed UG (haftungsbeschränkt), 95448 Bayreuth (DE)
(72) Erfinder: Rösch, Paul, 95445 Bayreuth (DE); Weiglmeier, Philipp, 95447 Bayreuth (DE); Berkner, Hanna, 79288 Gottenheim (DE)
(74) Vertreter: Hofstetter, Schurack & Partner

(57) **Zusammenfassung**

1. Verfahren zur rekombinanten Herstellung von mehrfach Disulfid-verbrückten Peptiden.

2.1. Disulfid-verbrückte Peptide werden bisher meist durch chemische Peptidsynthese dargestellt. Die Disulfidbrücken werden dabei meist unter Einsatz einer anspruchsvollen Schutzgruppenchemie geknüpft was die Ausbeute in vielen Fällen stark limitiert. Beiprodukte und inaktive Konformere lassen sich anschießend nur schwer vom Produkt trennen. Das neue Verfahren erlaubt die effiziente, rekombinante Darstellung von Disulfid-verbrückten Peptiden. Dabei wird die korrekte Faltung des Peptids und die Ausbildung nur der richtigen Disulfidbrücken durch eine aminoterminale Faltungshelfer-Sequenz unterstützt.

2.2. Die Pro-Sequenz von humanem Prouroguanylin, dem Vorläufer für das natriuretisch wirkende Hormon Uroguanylin, wird verwendet um das artfremde, bakterielle Enterotoxin STh rekombinant zu exprimieren. Die aminoterminale Pro-Sequenz ist als Faltungshelfer für die korrekte Ausbildung der drei Disulfidbrücken im carboxyterminalen STh verantwortlich. STh wird proteolytisch aus dem Fusionsprotein freigesetzt und über HPLC gereinigt. Auf diesem Weg wird die Bildung falsch Disulfid-verbrückter und biologisch inaktiver Konformere unterdrückt und STh kann rein erhalten werden.

2.3. Herstellung von STh, Herstellung anderer Cystein-reicher Peptide die als medizinische Therapeutika und Diagnostika von Interesse sind, z. B. Conotoxine.

## Beschreibung

**Die vorliegende Erfindung betrifft** die rekombinante Herstellung von mehrfach Disulfid-verbrückten Peptiden eines Organismus, gezeigt am Beispiel des hitzestabilen *E. coli* Enterotoxins STh, im präparativen Maßstab durch Verwendung eines Propetides aus einem anderen Organismus (hier: human).

Proteine und Peptide sind Polymere der natürlich vorkommenden Aminosäuren. Die Funktion dieser Biopolymere beispielsweise als Enzyme oder Signalstoffe setzt im Allgemeinen eine bestimmte dreidimensionale Faltung der Aminosäurekette voraus. Die dreidimensionale Struktur hängt hierbei wesentlich von der Sequenz der Aminosäuren in der Kette ab. Besonders schwierig ist der Übergang der zufällig strukturierten Aminosäurekette in ein definiert gefaltetes Biopolymer, wenn mehrere Disulfidbrücken in der Endstruktur vorkommen, da diese definiert zwischen je zwei spezifischen Aminosäuren aufgebaut werden müssen. Eine Disulfidbrücke besteht aus zwei über ihre Schwefelatome verknüpften Cystein-Aminosäuren. Beispielsweise können sich bei zwei Disulfidbrücken (4 Cysteine) insgesamt drei verschiedene Verbrückungsmuster ausbilden, von denen allerdings nur eines in einem biologisch aktiven Biopolymer resultiert.

Die Proteinfaltung ist ein äußerst komplexer Vorgang, der in der Natur durch sogenannte Faltungshelferproteine (Chaperone) oder intramolekulare Hilfssequenzen (Propeptide) unterstützt wird. Ohne diese Unterstützung kann es bei Proteinen beispielsweise durch vorzeitigen Abbau, Fehlfaltung oder Aggregation zur Bildung inaktiver Konformere kommen [1]. Chaperone und Propeptidsequenzen stellen sicher, dass solche Nebenreaktionen unterdrückt werden und die Zielsubstanz ausschließlich in der biologisch aktiven Konformation hergestellt wird.

Viele bakterielle und tierische Toxine, aber auch körpereigene Peptidhormone sowie eine große Zahl an extrazellulären Proteinen zeichnen sich durch einen hohen Gehalt an Cysteinen und eine charakteristische Disulfidverbrückung aus [2,3]. Das von bestimmten pathogenen *Escherichia* coli-Stämmen (ETEC = *enterotoxigenic E. coli*) exprimierte hitzestabile Enterotoxin STh enthält drei Disulfidbrücken. Diese sind für seine besonders starke Bindung an den physiologischen Rezeptor verantwortlich, die in intestinalen Epithelzellen exprimierte Guanylyl-Cyclase C (GC-C) [4-6]. GC-C ist ein Transmembran-Rezeptor, der nach Bindung eines Liganden an seine extrazelluläre Domäne die intrazelluläre Bildung von cyclischem GMP (cGMP) katalysiert. Dies setzt eine Signal-kaskade in Gang, die schließlich zur Sekretion von Elektrolyten in das Lumen des Darmes führt. Da STh einerseits als Verursacher vieler Formen von akuter sekretorischer Diarrhoe identifiziert wurde [7,8] und andererseits GC-C als bekannter Marker für kolorektale Karzinome dient [9-13], ist die Darstellung von biologisch aktivem STh von hohem pharmakologischen Interesse.

Durch chemische Peptidsynthese lassen sich kürzere Polypeptide effizient herstellen, jedoch sinkt die Ausbeute mit steigender Länge der Polypeptidkette erheblich. Entscheidend für den Syntheseerfolg sind weiterhin Parameter wie die Hydrophobizität und der Gehalt an weiteren Funktionalitäten, die in der Regel durch sterisch sehr anspruchsvolle Schutzgruppen vor unerwünschten Reaktionen geschützt werden. Diese Schutzgruppen werden nach Beendigung der Synthese abgespalten, um das Endprodukt zu erhalten. Um die richtige Disulfidverbrückung zu erhalten, müssen nochmals komplexere Synthesestrategien angewendet werden. Um beispielsweise gezielt zwei verschiedene Disulfidbrücken knüpfen zu können, müssen die Cysteine jeweils paarweise mit orthogonaler Schutzgruppenchemie versehen werden, die eine gezielte, einzelne Entschützung erlauben, sodass jeweils nur die beiden gewünschten Cysteine miteinander reagieren können [14].

**Nachteilig an diesem bekannten Ansatz** sind die oft stark limitierten Ausbeuten, die durch sterische Hinderung, unerwünschte Kreuzreaktionen von Cysteinen und andere Faktoren bedingt sind [15]. Zusätzlich ist es oft schwierig, das biologisch aktive Molekül von den häufig zahlreichen Nebenprodukten (Kettenabbruch, teilweise entschützt, falsche Verknüpfungen, etc.) zu trennen. Dies sind wesentliche Nachteile des Stands der Technik. Die rekombinante Herstellung von Proteinen durch Expression in geeigneten Bakterienstämmen oder Zelllinien schafft hier Abhilfe.

Zur rekombinanten Herstellung Disulfid-verbrückter Peptide und Proteine werden im Wesentlichen zwei Strategien angewendet. Durch Deletion spezifischer Reduktasen kann in einigen Organismen ein oxidatives Milieu im Cytoplasma geschaffen werden [16,17]. Dieses ermöglicht die Ausbildung von Disulfidbrücken in intrazellulär exprimierten Proteinen. Als Beispiel für solche Wirtsorganismen dienen die *E. coli* Stämme Origami B und AD494.

Eine zweite Möglichkeit zur Herstellung Disulfid-verbrückter Proteine besteht darin, das exprimierte Protein von einem Wirtsorganismus ins Periplasma (bei Bakterien) oder ins Kulturmedium sekretieren zu lassen. Dies wird erreicht, indem das Protein mit einer spezifischen Sekretions-Signalpeptidsequenz ausgestattet wird. Die Signalpeptidsequenz wird während des Exports zumeist abgespalten und die Disulfidbrücken können sich im oxidativen Milieu des Periplasmas bzw. des Kulturmediums bilden [18].

Für beide Methoden zur rekombinanten Herstellung von Disulfid-verbrückten Proteinen gilt, dass sie sich nur in Fällen eignen, in denen sich die Disulfidbrücken spontan in der richtigen Konformation ausbilden. Bei komplexeren Faltungstypen und insbesondere, wenn mehrere Disulfidverbrückungen ausgebildet werden müssen, können auch diese Methoden an ihre Grenzen stoßen.

Es ist daher **Aufgabe der vorliegenden Erfindung** die Herstellung von Proteinen mit komplizierten Disulfidverbrückungen zu ermöglichen. Dafür eignet sich die hier beschriebene Methode der Expression des Zielpeptids mit einem Propeptid, welches wie ein Chaperon wirkt. Wir zeigen, dass Propeptidsequenzen eine unerwartet breite Substratspezifität aufweisen können, wie dies auch für klassische Chaperone bekannt ist.

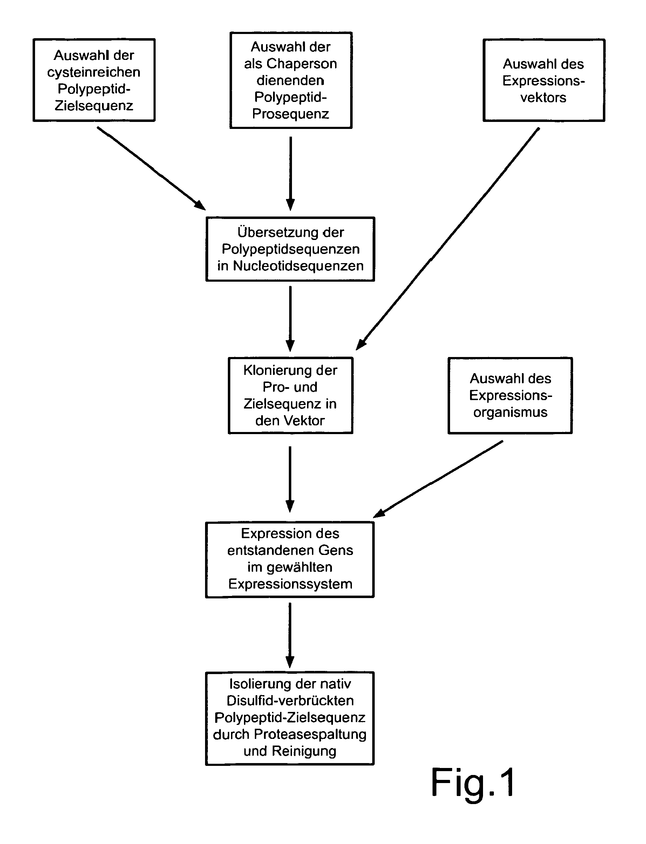

**Zur Lösung dieser Aufgabe** dient eine gattungsgemäße Vorrichtung gemäß den Merkmalen des Hauptanspruchs 1. Vorteilhafte Ausgestaltungen sind in den Unteransprüchen beschrieben. Abb. 1 (Anhang) stellt die hier vorgestellte Strategie schematisch als Flussdiagramm dar. Weitere Einzelheiten, Merkmale und Vorteile der Erfindung ergeben sich aus einem im **Folgenden dargestellten Ausführungsbeispiel.**

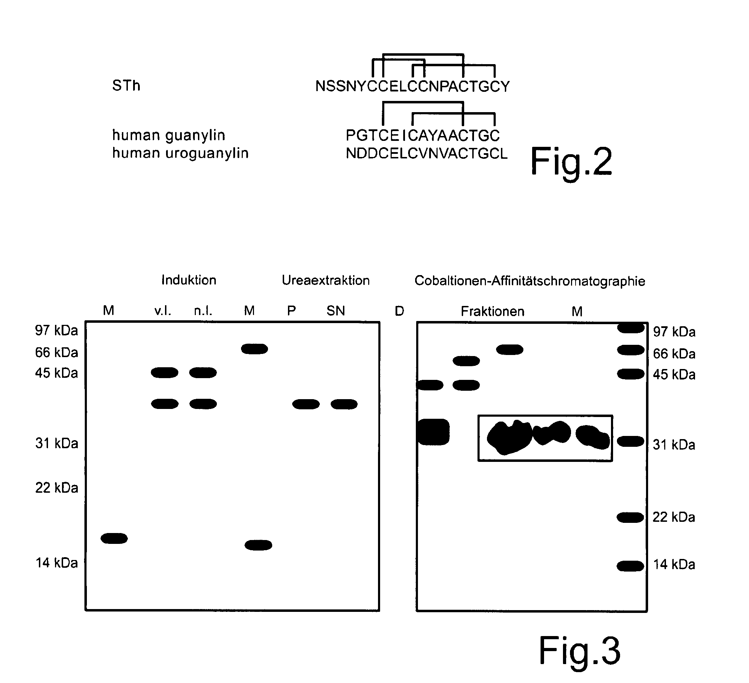

Die Peptidsequenzen der humanen Peptidhormone Guanylin und Uroguanylin, der körpereigenen Liganden von GC-C, und des bakteriellen Peptidtoxins STh unterscheiden sich (Anhang, Abb. 2). STh enthält im Vergleich zu den endogenen Peptiden eine zusätzliche Disulfid-Bindung.

Von Guanylin und Uroguanylin ist bekannt, dass die Faltung der Hormonsequenz und damit die richtige Ausbildung der beiden Disulfidbrücken maßgeblich von einer aminoterminalen Prosequenz abhängig ist [19,20]. Das aktive Hormon wird durch enzymatische Spaltung aus dem Prohormon freigesetzt. Für die rekombinante Herstellung von STh wurde ein Konstrukt erfunden, welches aus der aminoterminalen Proregion von Uroguanylin und der Sequenz von STh besteht. Die DNA-Sequenzen, die für die Proregion von Uroguanylin und für STh codieren wurden als synthetische Gene hergestellt. Anschließend wurde die Sequenz von STh 3'-terminal an die Proregion kloniert, an jene Position an der sich im nativen Prouroguanylin die für das Uroguanylin-Peptid codierende Sequenz befindet. Dieses Konstrukt, dessen Genprodukt im Folgenden als ProuroSTh bezeichnet wird, wurde in den *E. coli* Expressionsvektor pET32a (Merck, Darmstadt) kloniert. pET32a kodiert für einen aminoterminalen Hexahistidin- und Thioredoxin-Anhang, der die Löslichkeit verbessert und die Reinigung erleichtert. Die Expression erfolgte in *E. coli* Origami B (DE3). Dieser Stamm ermöglicht aufgrund seines oxidativen Cytoplasmas die intrazelluläre Bildung von Disulfidbrücken. Die Induktion der Proteinexpression erfolgte in Anwesenheit von IPTG (0,1 - 1 mM) für 14-18 h bei Temperaturen zwischen 15 °C und 37 °C. Nach der Lyse der Zellen wurde das Zielprotein, also die Thioredoxin-ProuroSTh-Fusion und mit konzentriertem Harnstoff (6 - 8 M) aus den Lysepellets extrahiert. Das Fusionsprotein wurde zurückgefaltet indem die Harnstoff-Konzentration durch Verdünnung auf 2 M gesenkt wurde. Anschließend wurde das Fusionsprotein über Cobaltionen-Affinitätschromatographie gereinigt (Anhang, Abb. 3). Der Hexahistidin-Thioredoxin-Anhang wurde mit Prescission-Protease abgespalten [21]. Das reine ProuroSTh wurde mit Trypsin verdaut. Die dabei entstehenden Fragmente wurden mittels Umkehrphasen-HPLC getrennt.

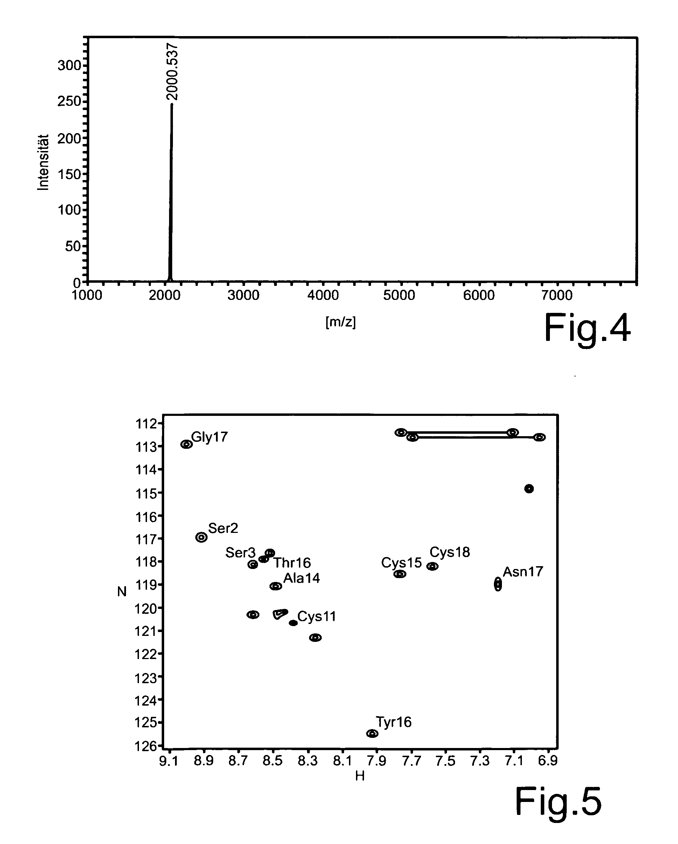

Die Identität des erhaltenen STh-Peptids und die vollständige Ausbildung der drei Disulfidbrücken wurde mittels Massenspektrometrie nachgewiesen (Anhang, Abb. 4). Zur Kontrolle wurden die Disulfidbrücken des erhaltenen STh mittels Dithiothreitol reduziert, was im Massenspektrum zu einer um 6 Da erhöhten Masse führte und somit die Ausbildung von drei Disulfidbrücken bestätigt. NMR-spektroskopische Untersuchungen ergaben für das bakterielle Toxin STh die native 3D-Konformation (Anhang, Abb. 5).

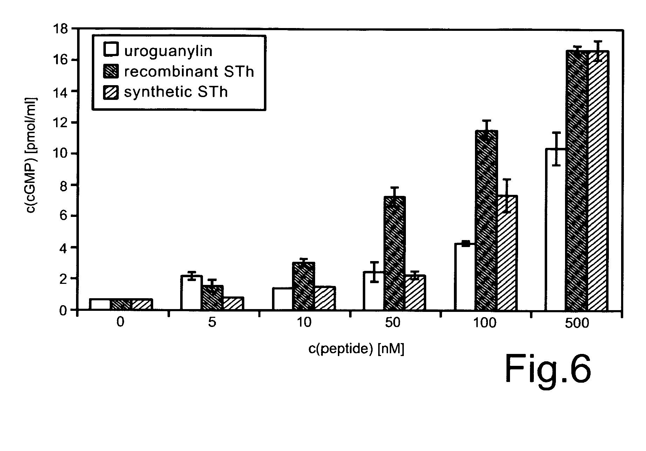

Die biologische Aktivität des so gereinigten Peptids wurde in einem *in-vivo-*Aktivitätstest bestätigt. Dabei wurde der in T84-Darmepithelzellen auftretende intrazelluläre cGMP-Anstieg nach der Zugabe und Bindung von STh an die von den Zellen exprimierte GC-C detektiert. Als Kontrollen dienten sowohl chemisch synthetisiertes STh als auch Uroguanylin (Anhang, Abb. 6).

Die **wesentliche Neuheit der hier vorgestellten Erfindung** besteht in der Verwendung eines Propeptids eines Organismus, das eingesetzt wird, um die biologisch aktive Form eines cysteinreichen Peptids eines anderen Organismus mit komplexem Disulfidverbrückungsmuster zu erhalten oder um die biologisch aktive Form eines cysteinreichen, nicht-nativen Peptids des ersten Organismus mit komplexem Disulfidverbrückungsmuster zu erhalten. Dabei wird die Fähigkeit der Proregion ausgenutzt, ein carboxyterminales Peptid selektiv in die jeweilige aktive Konformation zu falten und die Ausbildung der korrekten Disulfidbrücken zu unterstützen. Da sich die Peptidsequenzen von humanem Uroguanylin und bakteriellem STh deutlich unterscheiden, u. a. durch eine zusätzliche Disulfidbrücke in STh, kann von einer breiten Spezifität der hier verwendeten Proregion von Uroguanylin ausgegangen werden.

Gegenüber einer chemischen Synthese bietet das rekombinante Herstellungsverfahren eine nahezu uneingeschränkte Skalierbarkeit und somit die einfache Möglichkeit zur Herstellung großer Mengen an biologisch aktivem reinem Peptid. Unter anderem können auf diese Weise auch Isotopenmarkierungen einfach durchgeführt werden. Die Produktion von STh und verwandten Peptidhormonen und Toxinen ist insofern von eminentem Interesse, als diese Substanzen selbst und sequenzverwandte Analoga als pharmazeutische Wirkstoffe dienen. So werden Uroguanylin- und STh-Analoga als potentielles Abführmittel getestet [23,24]. Weiterhin werden Uroguanylin und STh sowohl als Marker als auch als potentielles Therapeutikum für Darmkrebs gehandelt [12,25-28].

Die vorgestellte Erfindung ist nicht abhängig vom Einsatz eines bestimmten Expressionsvektors oder -organismus. Es kann prinzipiell jedes beliebige bakterielle oder eukaryotische Proteinexpressionssystem verwendet werden, das die Ausbildung von Disulfidbrücken im exprimierten Protein erlaubt. Beim Einsatz anderer Expressionssysteme, beispielsweise mit anderen Expressiontags oder/und anderen Erkennungsregionen für Proteasen, ist gegebenenfalls die Proteinreinigungsstrategie an die spezifischen Gegebenheiten des Konstrukts anzupassen.

Die hier beschriebene Strategie zur Herstellung von STh ist übertragbar auf die Verwendung des Propeptids von Guanylin, dem zweiten endogenen Liganden von GC-C. Dies wird aus der hohen Peptidsequenzhomologie der beiden Propeptide abgeleitet. Gleichzeitig lässt die gezeigte breite Spezifität der Proregion von Uroguanylin den Einsatz der Methode bei der Darstellung anderer cysteinreicher Peptide zu, seien dies Uroguanylin- und STh-Analoga oder Peptide mit ähnlichem Faltungstyp.

Solche Moleküle spielen eine herausragende Rolle bei endokrinen und parakrinen Signaltransduktionsvorgängen im Körper, da sie häufig als Liganden für Transmembranrezeptoren fungieren. Beispiele hierfür sind neben den natriuretischen Peptiden Uroguanylin und Guanylin die sog. Endotheline, die als Vasokonstriktoren wirken und an der Regulation von Blutdruck und Gewebedurchblutung beteiligt sind.

Aufgrund ihrer meist hohen biologischen Aktivität fungieren viele cysteinreiche Peptide als Toxine. In analoger Weise wie das bakterielle Toxin STh zu einer Überaktivierung von GC-C führt, wirken auch tierische Toxine häufig aufgrund ihrer sehr festen Bindung an bestimmte Rezeptoren. Als Beispiele seien hier die in bestimmten Schlangengiften enthaltenen Sarafotoxine und die von Kegelschnecken produzierten Conotoxine genannt. Sarafotoxine sind Analoga der Endotheline und wirken kardiotoxisch, während viele Conotoxine an lonenkanäle und Rezeptoren binden, die an der neuronalen Signaltransduktion beteiligt sind [29-31].

Ihre hohe Spezifität und Aktivität macht viele tierische Toxine für die Verwendung als Wirkstoffe interessant. Jedoch bereitet ihre Verfügbarkeit häufig Schwierigkeiten, da Extraktion aus den natürlichen Quellen nicht praktikabel ist und ihre chemische oder rekombinante Herstellung den einleitend genannten Beschränkungen unterliegt. Die von uns beschriebene Erfindung ermöglicht die rekombinante Herstellung solcher komplex Disulfid-verbrückter Peptide. Sie zeichnet sich dadurch aus, dass die Stoffe rein, also nur in der aktiven Konformation, sowie in großen Maßstäben gewonnen werden können.

### Literatur:

1. Eder, J.; Fersht, A.R. Pro-Sequence-Assisted Protein Folding. Mol. Microbiol. 1995, 16, 609-614.
2. Azam, L.; Mclntosh, J.M. Alpha-Conotoxins as Pharmacological Probes of Nicotinic Acetylcholine Receptors. Acta Pharmacol. Sin. 2009, 30, 771-783.
3. Taylor, K.; Barran, P.E.; Dorin, J.R. Structure-Activity Relationships in Beta-Defensin Peptides. Biopolymers 2008, 90, 1-7.
4. Yoshimura, S.; Ikemura, H.; Watanabe, H.; Aimoto, S.; Shimonishi, Y.; Hara, S.; Takeda, T.; Miwatani, T.; Takeda, Y. Essential Structure for Full Enterotoxigenic Activity of Heat-Stable Enterotoxin Produced by Enterotoxigenic Escherichia Coli. FEBS Lett. 1985, 181, 138-142.
5. Gariepy, J.; Judd, A.K.; Schoolnik, G.K. Importance of Disulfide Bridges in the Structure and Activity of Escherichia Coli Enterotoxin ST1 b. Proc. Natl. Acad. Sci. U. S. A. 1987, 84, 8907-8911.
6. Weiglmeier, P.R.; Rösch, P.; Berkner, H. Cure and Curse: E. Coli Heat-Stable Enterotoxin and its Receptor Guanylyl Cyclase C. Toxins 2010, 2, 2213-2229.
7. Sack, R.B. Human Diarrheal Disease Caused by Enterotoxigenic Escherichia Coli. Annu. Rev. Microbiol. 1975, 29, 333-353.
8. Future Directions for Research on Enterotoxigenic Escherichia Coli Vaccines for Developing Countries. Wkly. Epidemiol. Rec. 2006, 81, 97-104.
9. Carrithers, S.L.; Barber, M.T.; Biswas, S.; Parkinson, S.J.; Park, P.K.; Goldstein, S.D.; Waldman, S.A. Guanylyl Cyclase C is a Selective Marker for Metastatic Colorectal Tumors in Human Extraintestinal Tissues. Proc. Natl. Acad. Sci. U. S. A. 1996, 93, 14827-14832.
10. Schulz, S.; Hyslop, T.; Haaf, J.; Bonaccorso, C.; Nielsen, K.; Witek, M.E.; Birbe, R.; Palazzo, J.; Weinberg, D.; Waldman, S.A. A Validated Quantitative Assay to Detect Occult Micrometastases by Reverse Transcriptase-Polymerase Chain Reaction of Guanylyl Cyclase C in Patients with Colorectal Cancer. Clin. Cancer Res. 2006, 12, 4545-4552.
11. Wolfe, H.R.; Mendizabal, M.; Lleong, E.; Cuthbertson, A.; Desai, V.; Pullan, S.; Fujii, D.K.; Morrison, M.; Pither, R.; Waldman, S.A. In Vivo Imaging of Human Colon Cancer Xenografts in Immunodeficient Mice using a Guanylyl Cyclase C--Specific Ligand. J. Nucl. Med 2002, 43, 392-399.
12. Giblin, M.F.; Sieckman, G.L.; Watkinson, L.D.; Daibes-Figueroa, S.; Hoffman, T.J.; Forte, L.R.; Volkert, W.A. Selective Targeting of E. Coli Heat-Stable Enterotoxin Analogs to Human Colon Cancer Cells. Anticancer Res. 2006, 26, 3243-3251.
13. Pitari, G.M.; Zingman, L.V.; Hodgson, D.M.; Alekseev, A.E.; Kazerounian, S.; Bienengraeber, M.; Hajnoczky, G.; Terzic, A.; Waldman, S.A. Bacterial Enterotoxins are Associated with Resistance to Colon Cancer. Proc. Natl. Acad. Sci. U. S. A. 2003, 100, 2695-2699.
14. Moroder, L.; Musiol, H.J.; Gotz, M.; Renner, C. Synthesis of Single- and Multiple-Stranded Cystine-Rich Peptides. Biopolymers 2005, 80, 85-97.
15. Houghten, R.A. SYNTHETIC HEAT-STABLE ENTEROTOXIN POLYPEPTIDE OF ESCHERICHIA COLI AND MULTIMERS THEREOF. *Patent* WO001984002700A1. 1983 Dec 21.
16. Stewart, E.J.; Aslund, F.; Beckwith, J. Disulfide Bond Formation in the Escherichia Coli Cytoplasm: An in Vivo Role Reversal for the Thioredoxins. EMBO J. 1998, 17, 5543-5550.
17. Bessette, P.H.; Aslund, F.; Beckwith, J.; Georgiou, G. Efficient Folding of Proteins with Multiple Disulfide Bonds in the Escherichia Coli Cytoplasm. Proc. Natl. Acad. Sci. U. S. A. 1999, 96, 13703-13708.
18. Georgiou, G.; Segatori, L. Preparative Expression of Secreted Proteins in Bacteria: Status Report and Future Prospects. Curr. Opin. Biotechnol. 2005, 16, 538-545.
19. Lauber, T.; Neudecker, P.; Rosch, P.; Marx, U.C. Solution Structure of Human Proguanylin: The Role of a Hormone Prosequence. J. Biol. Chem. 2003, 278, 24118-24124.
20. Lauber, T.; Marx, U.C. Prosequence-Mediated Disulfide Coupled Folding of the Peptide Hormones Guanylin and Uroguanylin. Protein Pept Lett. 2005, 12, 153-158.
21. Walker, P.A.; Leong, L.E.; Ng, P.W.; Tan, S.H.; Waller, S.; Murphy, D.; Porter, A.G. Efficient and Rapid Affinity Purification of Proteins using Recombinant Fusion Proteases. Biotechnology (N. Y) 1994, 12, 601-605.
22. Matecko, I.; Burmann, B.M.; Schweimer, K.; Kalbacher, H.; Einsiedel, J.; Gmeiner, P.; Rösch, P. Structural Characterization of the E. Coli Heat Stable Enterotoxin STh. Open Spectrosc J 2008, 2, 34-39.
23. Bryant, A.P.; Busby, R.W.; Bartolini, W.P.; Cordero, E.A.; Hannig, G.; Kessler, M.M.; Pierce, C.M.; Solinga, R.M.; Tobin, J.V.; Mahajan-Miklos, S. et al. Linaclotide is a Potent and Selective Guanylate Cyclase C Agonist that Elicits Pharmacological Effects Locally in the Gastrointestinal Tract. Life Sci. 2010, 86, 760-765.
24. Eutamene, H.; Bradesi, S.; Larauche, M.; Theodorou, V.; Beaufrand, C.; Ohning, G.; Fioramonti, J.; Cohen, M.; Bryant, A.P.; Kurtz, C. et al. Guanylate Cyclase C-Mediated Antinociceptive Effects of Linaclotide in Rodent Models of Visceral Pain. Neurogastroenterol. Motil. 2010, 22, 312-e84.
25. Pitari, G.M.; Di Guglielmo, M.D.; Park, J.; Schulz, S.; Waldman, S.A. Guanylyl Cyclase C Agonists Regulate Progression through the Cell Cycle of Human Colon Carcinoma Cells. Proc. Natl. Acad. Sci. U. S. A. 2001, 98, 7846-7851.
26. Giblin, M.F.; Gali, H.; Sieckman, G.L.; Owen, N.K.; Hoffman, T.J.; Volkert, W.A.; Forte, L.R. In Vitro and in Vivo Evaluation of 111In-Labeled E. Coli Heat-Stable Enterotoxin Analogs for Specific Targeting of Human Breast Cancers. Breast Cancer Res. Treat. 2006, 98, 7-15.
27. Li, P.; Lin, J.E.; Snook, A.E.; Gibbons, A.V.; Zuzga, D.S.; Schulz, S.; Pitari, G.M.; Waldman, S.A. Colorectal Cancer is a Paracrine Deficiency Syndrome Amenable to Oral Hormone Replacement Therapy. Clin. Transl. Sci. 2008, 1, 163-167.
28. Liu, D.; Overbey, D.; Watkinson, L.D.; Daibes-Figueroa, S.; Hoffman, T.J.; Forte, L.R.; Volkert, W.A.; Giblin, M.F. In Vivo Imaging of Human Colorectal Cancer using Radiolabeled Analogs of the Uroguanylin Peptide Hormone. Anticancer Res. 2009, 29, 3777-3783.
29. Sokolovsky, M. Endothelins and Sarafotoxins: Receptor Heterogeneity. Int J. Biochem. 1994, 26, 335-340.
30. Ducancel, F. Endothelin-Like Peptides. Cell Mol. Life Sci. 2005, 62, 2828-2839.
31. Heinemann, S.H.; Leipold, E. Conotoxins of the O-Superfamily Affecting Voltage-Gated Sodium Channels. Cell Mol. Life Sci. 2007, 64, 1329-1340.

## Patentansprüche

1. Verfahren zum Herstellen eines Zielpolypeptids, welches durch Biosynthese in einem natürlichen Organismus erster Art herstellbar ist, unter Verwendung eines Propeptids, welches bei einer Biosynthese eines anderen Polypeptids in einem natürlichen Organismus der ersten oder anderen Art zunächst aufgrund einer ersten DNA-Sequenz zusammen mit diesem anderen Polypeptid unter Bildung eines Fusionspolypeptids hergestellt wird, um die Faltung des anderen Polypeptids zu befördern, und nachfolgend von dem anderen Polypeptid abgetrennt wird, wobei
- eine zweite DNA-Sequenz, die für das Zielpolypeptid codiert, mit einem Teil der ersten DNA-Sequenz, der für das Propeptid codiert, zu einem Konstrukt gekoppelt wird,
- das Konstrukt in einen Vektor eingeführt wird,
- der Vektor in einen natürlichen Organismus eingebracht wird,
- der natürliche Organismus dabei gefördert wird, das Konstrukt zu exprimieren und ein so entstandenes Fusionspolypeptid, das das Zielpolypeptid und das Propeptid umfasst, zu speichern oder in ein anderes Medium abzugeben,
- aus dem Organismus bzw. dem anderen Medium das Fusionspolypeptid isoliert wird und
- das Propeptid von dem Zielpolypeptid abgetrennt wird.

2. Verfahren nach Anspruch 1, eingesetzt zum Herstellen eines Zielpolypeptids, das zumindest eine Disulfidbrücke aufweist.

3. Verfahren nach Anspruch 2, bei dem ein Propeptid verwendet wird, das bei der Herstellung eines solchen anderen Polypeptids durch Biosynthese hergestellt wird, das ebenfalls Disulfidbrücken oder mindestens ein Cystein aufweist.

4. Verfahren nach Anspruch 3,
**dadurch gekennzeichnet, dass**
das andere Polypeptid weniger Disulfidbrücken oder Cysteine aufweist als das Zielpolypeptid.

5. Verfahren nach einem der vorhergehenden Ansprüche,
**dadurch gekennzeichnet, dass**
das Zielpolypeptid ein Peptidhormon, insbesondere Guanylin, Uroguanylin, ein Hormon aus der Gruppe der Endotheline oder dgl., ein bakterielles Toxin, insbesondere das hitzestabile Enterotoxin STa aus *Escherichia coli,* ein Toxin aus einem höheren Organismus, insbesondere ein Conotoxin, Sarafotoxin, Chlorotoxin oder dgl., oder ein Regulation- oder Signaltransduktionspeptid, oder ein Derivat der vorgenannten eines höheren Organismus ist oder umfasst.

6. Verfahren nach Anspruch 5,
**dadurch gekennzeichnet, dass**
es einen vorbestimmten natürlichen Organismus gibt, in dem sowohl das Zielpolypeptid als auch das andere Polypeptid als Liganden für einen physiologischen Rezeptor des natürlichen Organismus fungieren können.

7. Verfahren nach Anspruch 6,
**dadurch gekennzeichnet, dass**
das andere Polypeptid Prouroguanylin, Uroguanylin oder ein Derivat davon ist oder umfasst.

8. Verfahren nach Anspruch 6 oder 7,
**dadurch gekennzeichnet, dass**
das Zielpolypeptid das von *Escherichia coli*-Bakterien exprimierte Enterotoxin STh oder ein Derivat davon ist oder umfasst.

9. Verfahren nach Anspruch 6 oder 7,
**dadurch gekennzeichnet, dass**
das Zielpolypeptid Guanylin oder ein Derivat davon ist oder umfasst.

10. Verfahren nach einem der vorhergehenden Ansprüche,
**dadurch gekennzeichnet, dass**
der Vektor in einen solchen natürlichen Organismus eingebracht wird, der ein oxidatives Milieu im Cytoplasma ausweist, insbesondere vorbestimmte Reduktasen nicht umfasst.

11. Verfahren nach einem der Ansprüche 1 bis 9,
**dadurch gekennzeichnet, dass**
der Vektor in einen solchen natürlichen Organismus eingebracht wird, der entweder
a) ein Bakterium mit einem Periplasma ist, wobei das Bakterium das Fusionspolypeptid in das Periplasma sekretiert, oder
b) der das Fusionspolypeptid in ein Kulturmedium sekretiert.

12. Verfahren nach einem der vorhergehenden Ansprüche,
**dadurch gekennzeichnet, dass**
als Vektor ein Plasmid, Cosmid, Phagemid, künstliches Bakterienchromosom, künstliches Hefechromosom oder ein Virus verwendet wird.

13. Verfahren nach einem der vorhergehenden Ansprüche,
**dadurch gekennzeichnet, dass**
der Vektor und/oder das Konstrukt wenigstens weitere DNA-Sequenz aufweist, die für einen Affinitätstag, einen Löslichkeits- und/oder Isoelektrizitätstag, eine Erkennungssequenz für ein weiteres Protein, insbesondere einen Epitoptag, einen Transport- oder Lokalisationstag, eine Proteaseerkennungssequenz, eine Zielsequenz für ein chemisches Spaltungsverfahren, insbesondere für die Bromcyanspaltung, oder eine Linkersequenz codiert.

14. Verfahren nach einem der vorhergehenden Ansprüche,
**dadurch gekennzeichnet, dass**
der mit dem Vektor versehene natürliche Organismus in einem Medium kultiviert wird, welches zumindest eine von dem natürlichen Organismus verwertbare Substanz enthält, die mit einem stabilen Isotop und/oder einem Radionuklid angereichert ist.

15. Verfahren nach einem der vorhergehenden Ansprüche,
**dadurch gekennzeichnet, dass**
der mit dem Vektor versehene natürliche Organismus in einem Medium kultiviert wird, welches zumindest eine von dem natürlichen Organismus verwertbare und in durch den Organismus exprimierte Polypeptide integrierbare Aminosäure mit zumindest einer Modifikation aus der Gruppe der Fluoreszenzmarker, paramagnetischen Marker, Chiralitätsmarker, Isotopenmarker oder der natürlichen posttranslationalen Modifikationen enthält.
